Europäisches Patentamt

(19) European Patent Office

Office européen des brevets

(11) Publication number: **0 051 955**

**B1**

(12) **EUROPEAN PATENT SPECIFICATION**

(45) Date of publication of patent specification: **24.04.85**

(51) Int. Cl.⁴: **A 61 F 2/02**

(21) Application number: **81305049.9**

(22) Date of filing: **26.10.81**

(54) **Porous body-implantable polytetrafluoroethylene composites.**

(30) Priority: **06.11.80 US 204528**

(43) Date of publication of application:
**19.05.82 Bulletin 82/20**

(45) Publication of the grant of the patent:
**24.04.85 Bulletin 85/17**

(84) Designated Contracting States:
**CH DE FR GB IT LI SE**

(56) References cited:
**US-A-3 992 725**
**US-A-4 118 532**

(73) Proprietor: **Homsy, Charles A.**
**11526 Raintree Cir.**
**Houston Texas 77024 (US)**

(72) Inventor: **Homsy, Charles A.**
**11526 Raintree Cir.**
**Houston Texas 77024 (US)**

(74) Representative: **Lambert, Hugh Richmond et al**
**D. YOUNG & CO. 10 Staple Inn**
**London, WC1V 7RD (GB)**

Courier Press, Leamington Spa, England.

## Description

The present invention relates to porous material for implantation into the human body and in which normal tissue growth may be fostered.

A variety of materials for implantation in the body and utilizing polytetrafluoroethylene (PTFE) as a base resin are known. PTFE is highly suitable for in vivo implantation due to its low incidence of rejection by the surrounding host tissue. However, it has been found that the intended site of implantation dictates the form of material to be used. Thus, some applications require supple thin sheets having low Young's modulus of elasticity, some require tubes of high flexibility and flexural fatigue resistance, while others require rigid, solid blocks of material that can be machined or carved. In other words, the human body comprises bone and tissue arranged in such a manner that different mechanical parameters apply in each application of the implantable material.

Consequently, there has been a continuous search for combinations of materials that can be utilized at specific sites in the body to accomplish a given set of tasks. Typical of these materials is the implantable material disclosed in U.S. Patent 3 992 725. Prior to that patent, there had not been a suitable, porous material providing for growth of normal body tissues in the implant. However, the ingrowth material obtained using implants according to that patent is usually black in color, due to the fibers used therein. In addition, the tensile stress behavior, abrasive resistance and flexural fatigue life of that material limits some of its potential uses.

Other work in this field is typified by U.S. Patent 3 556 161, directed to PTFE resin sheets.

The present invention, however, overcomes these and other deficiencies, as explained more completely hereinafter.

The present invention seeks to provide an improved material for in vivo implantation which is light in color, promotes ingrowth of body tissue and which has an improved abrasion resistance and flexural fatigue life.

According to the present invention an improved composite material for *in vivo* implantation is provided composed entirely of polytetrafluoroethylene (PTFE). More particularly the composite material of this invention comprises a porous, compacted and sintered blend of polytetrafluoroethylene fibers and polytetrafluoroethylene particles which act as a binder for the fibers. According to the invention, this composite is made by forming a blend consisting of polytetrafluoroethylene (PTFE) resin in particulate form, PTFE fibers and from about 50 to about 90 percent by volume of a particulate pore-forming material which is soluble in a non-solvent for said PTFE; forming a mat from said blend; compacting said mat; sintering said compacted mat at a temperature which fuses said PTFE particles and fibers into a continuous matrix; and subsequently dissolving out from said sintered, compacted mat substantially all of said pore-forming material. The porous composite material obtained generally has a porosity of from about 50 to about 90 percent by volume; a tensile strength of from 20 to about 300 Kg per cm$^2$; and for porosity above 75 percent has essentially no region of constant elastic modulus (Young's modulus). The major proportion of the pores, in the porous composite of the invention, have a pore size distribution of between about 80 to about 400 microns.

If desired the sheet material produced in accordance with this invention may be bonded to a layer of the implantable material described in U.S. Patent 3 992 725 to form a laminated composite.

It has been unexpectedly found that PTFE structures of this invention compensate for the relatively low surface energy of PTFE polymer by providing a pore structure of irregular geometry and enormous surface area. Apparently, the ability of cells to attach and move within the porous structures of this invention is greatly enhanced by the geometry of this structure which derives from a combination of polymer fibrous elements and polymer nodal elements. The present material, however, does not have the ingrowth potential of the material claimed in U.S. Patent 3 992 725 because it exhibits a lower surface energy than achieved with the latter material. However, laminates of the present invention with that material are part of the present invention.

In materials of the present invention the volume ratio of PTFE fiber to resin is preferred to be 1 to 1 for 80% porous structures and greater than 1 to 1 for less porous structures, e.g., increasing to 2.3 to 1 for 50% porous structures. The PTFE fibers are preferred to have a strand length up to 2 inches (5 cms.).

The preferred particulate pore-forming materials used in this invention are water-soluble compounds such as sodium chloride crystals, of particle size between 10 and 500 microns.

Alternatively other soluble material/solvent combinations may be used. For example, when water is to be the solvent, other water soluble salts which are thermally stable at temperatures below about 700°F (371°C) may be used such as sodium carbonate, calcium fluoride, or magnesium sulphate, and others. It will generally be preferred to use the sodium chloride-water system since the sodium chloride would be completely compatible in the body in the event small amounts were left in the material from the leaching step.

A typical formulation would include on a volume basis 80% sodium chloride, 10% PTFE resin fibers and 10% PTFE particulate resin. Such formulation when prepared as herein set forth has been found to have moderate issue ingrowth, sufficient strength and sufficient flexural properties. Table I shows the preferred range of components.

### TABLE I

| | |
|---|---|
| PTFE resin fibers | 2.5% to 35% by volume |
| PTFE resin particles | 2.5% to 25% by volume |
| soluble material | 90% to 50% by volume |

The steps involved in preparing these materials are as follows:

a. Mixing: In this step the PTFE polymer resin, PTFE fibers and soluble ingredients are suspended in a suitable organic solvent such as purified isoparaffinic solvent; it is preferred that aromatic content of such solvent be less than 1% by weight; the resulting slurry is mixed at very high speed in a high shear mixer such as a Waring blender. The proportion of solvent to dry ingredients is important and must be adjusted to the size of the mixer used. The total volume of our mixer is 1000 milliliters and 500 milliliters of solvent are used for dry ingredient weights of the order of 80 grams. Mixing is carried out for between one and five minutes depending upon the particular ingredients used. The fibers are approximately 1/4 to 3/8 inch (6 to 9.5 mm) long.

b. Filtration: The mixed slurry is rapidly poured into a vacuum filter such a Buchner funnel, and filtration proceeds from between a few seconds to several minutes depending upon ingredients used. The residual solvent left in the filter cake is carefully monitored so as to be less than about 20% by weight.

c. Compression: The filter cake from step (b) is placed within the platens of a heated press heated to 100° to 200°F (38° to 93°C) and compression is applied at levels of between 50 to 1,500 pounds per square inch (345 to 10350 KPa) for between one and five minutes, again depending upon the particular ingredients. The present residual solvent in the filter cake after compression is routinely monitored and conditions are adjusted so that the level of solvent is between six and twenty weight percent.

d. Rolling: The compressed filter cake from step (c) is run through the nip of heated rolls such that the thickness of the cake is reduced in decrements of approximately 20 mils (500 μm) to levels of from 80 to 20 mils (2000 to 500 μm) depending upon the intended final thickness of the product. The temperature of the heated rolls should be in the range of 100° to 280°F (38° to 138°C). That is, heated rolls over this temperature range are required in order to help volatilize the carrier solvent. Moreover, during this step each pass through the rolls is made perpendicular with the direction of the previous rolling maneuver.

e. Drying: Stock material is dried to evaporate any residual solvent by placing in an oven held at temperatures of between 150° and 350°F (66° and 177°C) for several hours—up to 48 hours usually.

f. Sintering: The dried stock is now sintered. Sintering is carried out in a heated press at temperatures between 625° and 680°F (329° and 360°C) and a pressure of from 500—750 p.s.i. (3448—5171 KPa) for periods of time from 4 to 15 minutes, or more, depending upon the thickness of the material being sintered. The mats are usually about 1 to 2 mm thick prior to sintering. In order to prepare material of varying thickness, a plurality of 1 to 2 mm mats are stacked, and then sintered. Sintering time depends upon the number of layers of mats and temperature, at a given pressure. The preferred pressure is from 550 to 675 psi (3792 to 4654 KPa), and most preferably at 625 p.s.i. (4309 KPa), the best temperature for sintering is about 650°F (343°C) for the times shown in Table II, below:

TABLE II—Sintering Times, at 650°F (343°C)

| Material thickness, mm | Time, minutes |
|---|---|
| 1.2 | 4—6 |
| 3 | 8 |
| 4 | 9 |
| 5 | 10 |
| 6—10 | 15 |

With two or more layers and with thickness greater than 1·mm, the preferred pressure will be approximately one-half the above stated pressure.

g. Leaching: The stock is leached to dissolve out water soluble filler material by placement in a container containing distilled water and thereby develop discrete volume and porosity. Distilled water may be caused to flow at a slow rate through such container in order to provide the maximum driving force for diffusion of dissolved filler from the stock in a leaching water. The leaching step is usually allowed to proceed for 48 hours for stock having a thickness of 5 mm—10 mm. Longer times would probably be required for thicker stock. The distilled water is preferred to be warm to increase the rate of dissolving of salt.

h. Drying: The leach stock is then placed in an oven held at a temperature between about 160° and 350°F (71° to 177°C) in order to affect drying of the residual water contained within the stock material. The drying step may include a 24 hour hold at 300°F (149°C) to volatilize any residual solvent.

The product material from the above series of steps exhibits several important properties of significance to tissue ingrowth. During the leaching step the voids are created in the material. A portion of the voids have a nodal shape as they are formed by the leaching of generally spheroidal sodium chloride crystals from the material. In addition, the material which is produced as described also develops dendritic voids which interconnect in random fashion with the spheroidal voids to thereby provide a particularly effective open structure for the ingrowth of cellular elements which develop and mature fibrous tissue within the voids. However, the surface free energy of the material and specific surface area are lower than in the material of U.S. Patent 3 992 725. As a result, a higher percentage of ingrowth occurs in the latter material than in the present. For some applications it is advisable to use a combination of materials having a first layer of the material of U.S. Patent 3 992 725 and a fused layer of the all-PTFE material of this invention. This is particularly true where the combination material is to be used close to the skin, of a person of light complexion. The material of the present invention is essentially a white color.

It is believed that with the development and maturation of tissue within such voids, such tissue is not as vulnerable to infection as prior implants since substantial blood supply is developed to allow the normal body functions for fighting infection to be active within such material. In prior materials for implants, the appearance of an infection in connection with an implanted device generally necessitated the removal of the device if the normal body infection fighting mechanisms were not able to reach the infected area.

Additionally, because of the resiliency and distensibility of this composition of material, the tissues developing therein feel or are subjected to the normal mechanical forces at the sites of the implant which assist in the formation of the type of tissue needed at such location.

Upon consideration of the promotion into the composition of material of ingrowth of body tissues, such composition of material appears to have varied applications in all parts of the body, e.g., soft tissue augmentation, partial and complete joint prostheses, birth control by vas or tube blockage from material implant, fixation of artificial teeth, tendon and ligament replacement and fixation, alveolar ridge augmentation and other implant procedures.

It has been found that the kind and porosity of the composition of material may be controlled by the amount of soluble filler included in the original mixture.

In the formation of the material the rolling proceeds until the thickness of the material is of the order of one to two millimeters (mm) thick to thereby provide a maximum strength. When thicker stock is desired particularly for soft tissue and alveolar ridge augmentation it can be achieved by following the above steps (a), (b), (c), (d) and (e) and then stacking the dried stock to the desired multiple of the single ply thickness. The stacked layers are sandwiched between aluminum foil and placed within the platens of a press held at a temperature of from 625° to 680°F (329°—360°C). Pressure is applied gradually depending on the area of the laminate to a final hold pressure of about 625 p.s.i. (4309 KPa). This hold pressure is maintained for a period of time as set out above and may not need to exceed fifteen minutes. This laminated stock is then leached and dried as set forth in steps (g) and (h) above.

When the mat has been compacted, sintering should preferably be done under conditions which allow the resin to gel and fuse the fibers together without substantially diminishing the higher tensile strength of the fibers. The tensile strength of the PTFE fibers, above, is approximately 10 times that of the PTFE resin alone.

Subsequent removal from the sintered, compacted mat of essentially all of the material which is soluble in a non-solvent for the PTFE results in a flexible material whose strength is substantially greater than that to be expected for PTFE resins.

Although the published gelation temperature for PTFE fibers is given as that of the resin, it has been unexpectedly found that under the conditions of preparation of this invention, gelation of resin which fuses the fibers into a continuous matrix does not impair the fibrous quality of the fibers themselves.

The preferred ingredients, useful in preparing the compositions of the present invention are bleached fibers of Teflon TFE and Teflon TFE-6 resin, sold by DuPont Company ("Teflon" is a Registered Trade Mark). The sodium chloride, reagent grade crystals, was supplied by J. T. Baker Laboratory Chemicals, but may be any equivalent grade supplied in the industry.

In preparing the all-TFE mats, there is mixed together the PTFE resin and fibers in a volume ratio between 1:5 and 10:1. The preferred composition for 80% porous structures has a ratio of resin to fiber of 1:1. This particular formulation is a relatively high strength, but exceedingly porous, material exhibiting ultimate tensile strength of the order of 40 kg/cm$^2$ and at the same time, providing essentially no region of constant elastic modulus (Young's modulus) as defined from the conventional stress-strain curve. That is, the all-TFE compositions are implant materials providing the low modulus advantage in not injuring ingrowth tissue. This is especially important with an all-TFE composition where the rate of ingrowth is lower than that observed with the carbon fiber/PTFE composition of U.S. Patent 3 992 725.

The all-TFE compositions of the present invention can be laminated to suitable metallic or other substrates which will withstand temperatures of fusion bonding using the bonding methods described fully in U.S. Patent 3 992 725. That is, by using as a bonding substrate a thin layer of fluorinated ethylenepropylene (such as sold by DuPont Company under the name Teflon FEP). Particularly suitable are laminations of the present invention to bio-compatible fabric such as polyamide, polyaramide, polyimide or polyester fabric. This, also, may be accomplished by the method of using the Teflon FEP resin, described above.

As already indicated, a particularly preferred form of laminate in accordance with this invention is a laminate comprising a layer or layers of an all-polytetrafluoroethylene composite in accordance with this invention laminated with a layer of the porous, biocompatible composite described in U.S. Patent 3 992 725. Broadly speaking this other layer may be defined as being comprised of a different or different blend of biocompatible fibers bonded with a biocompatible resin, said other layer having a porous structure and a critical surface tension in the range 35—80 gynes per centimetre. More specifically and preferably the same other layer is, as described in that patent, comprised of a fused blend of polytetrafluoroethylene fibers and carbon fibers bonded with a biocompatible polytetrafuluroethylene resin. Such other layer may be formed by substantially the same procedure as hereinbefore described, that is to say by forming a blend of the biocompatible fibers and the biocompatible resin, the blend also containing a pure forming agent, e.g. sodium chloride, forming the blend into a mat, compacting and sintering the mat, and leaching out the pore forming agent. Further and more specific details of this type of material may be had by reference to the aforementioned U.S. Patent. Preferably, in accordance with this invention, the porosity of the two layers is such that the proportion of pores having a spheroidal or denduitic configuration is greater in the substrate layer, i.e. the layer containing the carbon fibers, than the all-polytetrafluoroethylene layer in accordance with this invention.

Silicone rubber also may be used to form a lamination with the porous material of the present invention. Such lamination may be accomplished by any suitable method. For example dipping or spray coating a porous web of material according to the present invention with a dispersion of medical grade silicone rubber in a non-polar vehicle provides a tenaciously bounded coating on the porous material. Laminations of silicone rubber and the porous material of this invention are useful in sealing surface porosity and in bonding the porous material to other materials.

## Claims

1. A composite *in vivo* implantation material comprising a porous, compacted, sintered blend of biocompatible resin fibers and a biocompatible resin binder, characterized in that the fibre component consists entirely of fibres of polytetrafluoroethylene and in that the binder component consists entirely of particles of polytetrafluoroethylene resin fused together to form a porous matrix embodying said polytetrafluoroethylene fibers.

2. A material according to claim 1, characterized in that the volume ratio of resin to fiber in said blend is in the range 1:1 to 2.3:1.

3. A material according to claim 1 or 2 characterized by a porosity of from 60—80% by volume.

4. A material according to any one of the preceding claims, characterized in that the majority of the pores in the composite have a size in the range 80—400 microns.

5. A porous laminate suitable for *in vivo* implantation, characterised in that it comprises at least one layer of a porous composite material according to any one of the preceding claims.

6. A laminate according to Claim 5 characterised in that it comprises a plurality of layers of a material as claimed in any one of claims 1—4 bonded together.

7. A laminate according to claim 5 or 6, characterised in that it comprises at least one other layer which is comprised of a different or different blend of biocompatible fibers bonded with a biocompatible resin, said other layer having a porous structure and a critical surface tension in the range 35—80 dynes per centimetre.

8. A laminate according to claim 7, characterised in that said other layer is comprised of a fused blend of polytetrafluoroethylene fibers and carbon fibers bonded with a biocompatible polytetrafluoroethylene resin.

9. A laminate according to claim 7 or 8, characterised in that the said other layer has a greater proportion of porosity consisting of spheroidal and dendritic spaces than that of the said layer of material as claimed in any one of claims 1—4.

10. A laminate according to claim 5, characterised in that comprises a first layer of a material as claimed in any one of claims 1—4, and a second layer, bonded thereto, of a biocompatible silicone rubber.

11. An implant for *in vivo* implantation comprising a tube of a material as claimed in any one of claims 1—4 or a laminate according to any one of claims 5—10.

## Revendications

1. Un matériau composite d'implantation in vivo comprenant un mélange fritté compacté poreux de fibres de résine biocompatible et d'un liant résineux biocompatible, caractérisé en ce que les fibres sont constituées entièrement de fibres de polytétrafluoroéthylène et en ce que le liant est constitué entièrement de particules de résine de polytétrafluoroéthylène soudées ensemble pour former une matrice poreuse contenant lesdites fibres de polytétrafluoroéthylène.

2. Un matériau selon la revendication 1, caractérisé en ce que le rapport en volume de la résine aux fibres dans ledit mélange est dans la gamme de 1/1 à 2,3/1.

3. Un matériau selon la revendication 1 ou 2, caractérisé par une porosité de 60 à 80% en volume.

4. Un matériau selon l'une quelconque des revendications précédentes, caractérisé en ce que la majorité des pores du composite a une taille dans la gamme de 80—400 microns.

5. Un stratifié poreux convenant pour l'implantation in vivo, caractérise en ce qu'il comprend au moins une couche d'un matériau composite poreux selon l'une quelconque des revendications précédentes.

6. Un stratifié selon la revendication 5, caractérisé en ce qu'il comprend plusieurs couches d'un matériau comme revendiqué dans l'une quelconque des revendications 1 à 4 unies ensemble.

7. Un stratifié selon la revendication 5 ou 6, caractérisé en ce qu'il comprend au moins une autre couche qui est constituée de fibres biocompatibles différentes ou d'un mélange de telles fibres différentes avec une résine biocompatible, ladite autre couche ayant une structure poreuse at une tension superficielle critique dans la gamme de 35—80 dynes par centimètre.

8. Un stratifié selon la revendication 7, caractérisé en ce que ladite autre couche est constituée d'une mélange soudé de fibres de polytétrafluoroéthylène et de fibres de carbone unies avec une résine de polytétrafluoroéthylène biocompatible.

9. Un stratifié selon la revendication 7 ou 8, caractérisé en ce que ladite autre couche a une proportion de porosité résultant d'espaces sphéroïdaux et dendritiques supérieure à celle de ladite couche de matériau comme revendiqué dans l'une quelconque des revendications 1 à 4.

10. Un stratifié selon la revendication 5, caractérisé en ce qu'il comprend une première couche d'une matériau comme revendique dans l'une quelconque des revendications 1 à 4, et une seconde couche, qui lui est unie, d'un caoutchouc de silicone biocompatible.

11. Un implant pour l'implantation in vivo comprenant un tube d'un matériau comme revendiqué dans l'une quelconque des revendications 1 à 4 ou d'une stratifié selon l'une quelconque des revendications 5 à 10.

**Patentansprüche**

1. Zusammengesetztes Material für in-vivo-Implantationen aus einem porösen, verdichteten, gesinterten Gemisch von Fasern aus biologisch verträglichem Harz und einem biologisch verträglichen Harzbindemittel, dadurch gekennzeichnet, daß die Faserkomponente vollständig aus Polytetra-fluoräthylenfasen besteht und daß die Bindemittelkomponente vollständig aus Polytetrafluoräthylenharzteilchen besteht, die unter Bildung einer die Polytetrafluoräthylenfasen einschließenden porösen Matrix miteinander verschmolzen sind.

2. Material nach Anspruch 1, dadurch gekennzeichnet, daß das Volumenverhältnis von Harz zu Fasern in dem Gemisch im Bereich von 1:1 bis 2,3:1 liegt.

3. Material nach Anspruch 1 oder 2, gekennzeichnet durch eine Porosität von 60 bis 80 Vol.-%.

4. Material nach einem der vorausgehenden Ansprüche, dadurch gekennzeichnet, daß der Hauptteil der Poren in der Zusammensetzung eine Größe im Bereich von 80 bis 400 Mikron besitzt.

5. Für eine in-vivo-Implantation geeignetes poröses Laminate, dadurch gekennzeichnet, daß es wenigstens eine Schicht eines porösen zusammengesetzten Materials nach einem der vorausgehenden Ansprüche aufweist.

6. Laminat nach Anspruch 5, dadurch gekennzeichnet, daß es mehrere miteinander verbundene Schichten eines Materials nach einem der Ansprüche 1 bis 4 aufweist.

7. Laminat nach Anspruch 5 oder 6, dadurch gekennzeichnet, daß es wenigstens eine andere Schicht aufweist, die andere biologisch verträgliche Fasern oder ein anderes Gemisch biologisch verträglicher Fasern, mit einem biologisch verträglichen Harz verbunden, umfaßt, wobei diese andere Schicht eine poröse Struktur und eine kritische Oberflächenspannung im Bereich von 35 bis 80 dyn je Zentimeter hat.

8. Laminat nach Anspruch 7, dadurch gekennzeichnet, daß die andere Schicht ein verschmolzenes Gemisch von . Polytetrafluoräthylenfasern und Kohlenstoffasern, die mit einem biologisch verträglichen Polytetrafluoräthylenharz verbunden sind, umfaßt.

9. Laminat nach Anspruch 7 oder 8, dadurch gekennzeichnet, daß die andere Schicht einen größeren Anteil an Porosität, die aus spheroidischen und dendritischen Hohlräumen besteht, als die Materialschicht gemäß einem der Ansprüche 1 bis 4 hat.

10. Laminat nach Anspruch 5, dadurch gekennzeichnet, daß es eine erste Schicht aus einem Material gemäß einem der Ansprüche 1 bis 4 und eine zweite daran gebundene Schicht aus einem biologisch verträglichen Silikonkautschuk umfaßt.

11. Implantat für in-vivo-Implantation mit einer Röhre aus einem Material nach einem der Ansprüche 1 bis 4 oder einem Laminat nach einem der Ansprüche 5 bis 10.